# EUROPEAN PATENT APPLICATION

(11) **EP 3 366 188 A1**
(43) Date of publication of application: **29.08.2018**
(21) Application number: 15906656.2
(22) Date of filing: 20.10.2015
(51) Int. Cl.: A61B 1/00, G02B 23/24

(54) **INSERTION BODY SUPPORT SYSTEM**

(71) Applicant: Olympus Corporation, Hachioji-shi, Tokyo 192-8507 (JP)
(72) Inventor: HANE, Jun, Tokyo 192-8507 (JP); FUJITA, Hiromasa, Tokyo 192-8507 (JP)
(74) Representative: Winter, Brandl, Fürniss, Hübner, Röss, Kaiser, Polte - Partnerschaft mbB
(86) International application number: PCT/JP2015/079575
(87) International publication number: WO 2017/068650

(57) **Abstract**

The insertion unit support system is provided for a tubular insertion system, and generates lumen information including the shape and location of a lumen based on the pre-acquired information about the lumen of a subject as an observation target that includes image information of two- or higher dimensional images, i.e., three-dimensional images or three-dimensional tomograms, so that the lumen information is used as support information for inserting the insertion unit of an endoscope. The lumens of the subjects targeted for the support of the insertion unit support system may vary in shape and location, and are deformed according to the shape of an inserted insertion unit. Thus, the support information is corrected and updated based on the deformation.

## Description

### FIELD

The present invention relates to an insertion unit support system for supporting operations of a tubular insertion system as typified by a flexible endoscope, a catheter, or the like that is adapted to be inserted into lumens for performing observation and treatment procedures such as repair, remedy, and sampling.

### BACKGROUND

An endoscope is generally known as one example of a tubular insertion system that performs observation, etc., while being inserted into any lumen or body cavity. An insertion portion (or insertion unit) of an endoscope, once inserted into a lumen, does not allow for direct visual recognition of its position or bending form. As such, skill and experience are required to determine the up and down orientations or the positional relationships to organs (body cavities) through the observation images taken by an endoscope inserted into an observation subject (patient).

Added to this, an observation target may be a large bowel, which is an organ varying in shape and location for each patient and which can be deformed according to the shape of an insertion portion. Thus, determining the insertion state (e.g., position in the large bowel, bending form, etc.) of an insertion portion in consideration of the shape and location of the large bowel, while looking at a screen displaying the obtained images, would largely depend on assumptions made based on operator's knowledge and experience. There is therefore a concern that without advice or some assistance from an expert, a less-skilled operator would face troubles when inserting or extracting an insertion portion and could take an unnecessarily long time. Besides, even an expert does not always make the right decision and might have to perform trial-and-error work.

### PATENT LITERATURE

### 1. Jpn. Pat. Appln. KOKAI Publication No. 2014-204904

### SUMMARY

### TECHNICAL PROBLEM

As discussed above, once an insertion portion of an endoscope, etc. is inserted into a body cavity, the shape of the insertion portion cannot be seen directly. Accordingly, a system has been proposed, in which an insertion portion is provided with a sensor for detecting position information, and the bending form is detected from the information about changes in optical intensity and amount acquired by the sensor. There is a further proposal of a system as in, for example, Jpn. Pat. Appln. KOKAI Publication No. 2014-204904 (Patent Literature 1) that pre-acquires and utilizes not only two-dimensional images from X-ray imaging as used in diagnosis, but also three-dimensional tomograms of an observation target from external imaging devices such as a computed tomography (CT) apparatus and a magnetic resonance imaging (MRI) apparatus. This Patent Literature 1 proposes an ultrasonic endoscope system utilizing CT images. The ultrasonic endoscope system generates subject model image data using CT images and body axis direction data, and combines insertion form data, an ultrasonic tomogram marker, a scope distal end marker, three-dimensional living tissue model data, the subject model image data, and derived image information to generate and display various guide images.

As in these proposals, approaches to improving insertability are being tried through the incorporation of a position sensor or a shape sensor into an insertion portion of an endoscope, etc. so that the position, form, posture, etc. of the insertion portion is detected and the information is provided to an operator.

However, a concrete implementation method that can realize practical use of two- or higher dimensional images, in particular, three-dimensional images (or three-dimensional tomograms) obtained as CT images, ultrasonic images, etc., in the endoscopic diagnosis for observation targets, such as a large bowel, varying in shape and location depending on a patient's individual difference and deformed according to the insertion state of an insertion unit, has yet to be proposed.

The object of the present invention is to provide an insertion unit support system that creates lumen information including lumen shape information from the pre-acquired two- or higher dimensional images or tomograms of a lumen as an observation target, and uses the lumen information as support information for inserting an insertion portion of an endoscope, etc. into the lumen.

### SOLUTION TO PROBLEM

According to an embodiment of the present invention, there is provided an insertion unit support system configured to provide support information for operations with an insertion unit adapted to be inserted into a given lumen of a subject, wherein the operations comprise insertion and extraction of the insertion unit, observation, and treatment, and wherein the support information is based on pre-acquired lumen information comprising two- or higher dimensional shape information for the lumen.

### ADVANTAGEOUS EFFECTS OF INVENTION

According to the present invention, it is possible to provide an insertion unit support system that creates lumen information including lumen shape information from the pre-acquired two- or higher dimensional images or tomograms of a lumen as an observation target, and uses the lumen information as support information for inserting an insertion portion of an endoscope, etc. into the lumen.

### BRIEF DESCRIPTION OF THE DRAWINGS

FIG. 1 is a diagram showing a configuration example of an insertion unit support system provided for a tubular insertion system according to one embodiment of the present invention.
FIG. 2 is a diagram showing external structures of an endoscope having the insertion unit support system.
FIG. 3 is a diagram showing bending directions of a bending portion at the distal end of an insertion portion of the endoscope.
FIG. 4 is a diagram showing external structures of a CT apparatus.
FIG. 5 is a conceptual diagram showing images of a subject taken from a specific viewpoint.
FIG. 6 is a diagram showing a concept of helical-scan imaging in a CT apparatus.
FIG. 7 is a diagram showing external structures of an MRI apparatus.
FIG. 8 is a diagram showing a diagnostic situation using an ultrasonic diagnosis apparatus.
FIG. 9 is a diagram showing external structures of an ultrasound diagnostic apparatus and probes for use.
FIG. 10 is a diagram showing a configuration example of a bending-form detector for detecting the bending form of an insertion unit.
FIG. 11A is a diagram showing detection light that is guided within an optical fiber bent in a direction of the side where a fiber sensor is attached.
FIG. 11B is a diagram showing detection light that is guided within an optical fiber in a straightened state.
FIG. 11C is a diagram showing detection light that is guided within an optical fiber bent in a direction opposite to the side where a fiber sensor is attached.
FIG. 12 is a diagram showing an example of setting an insertion-unit sensor at an opening of a subject's lumen.
FIG. 13A is a diagram showing a lumen information integrating processor furnished in a subject information extractor.
FIG. 13B is a diagram showing a position information integrating processor furnished in the lumen information integrating processor.
FIG. 13C is a diagram showing a lumen-associated information estimate generator furnished in the lumen information integrating processor.
FIG. 13D is a diagram showing a lumen location-associated information estimate generator furnished in the position information integrating processor.
FIG. 14 is a diagram showing lying states of a subject placed on an examination table.
FIG. 15 is a flowchart for explaining generation and output of operation support information in the insertion unit support system.
FIG. 16 is a flowchart for explaining processing to correct or update the information for a subject's lumen state.
FIG. 17 is a flowchart for explaining support information processing for presenting a subject's estimated lumen state and a subject's measured lumen state.
FIG. 18 is a diagram showing a certain example of the operation support information displayed on a display.
FIG. 19 is a diagram showing a screen that displays insertion-state detection, as a first display example.
FIG. 20 is a diagram showing a screen that displays insertion-state detection, as a second display example.
FIG. 21A is a diagram showing an instance in which a third display example is formed, in which presentation and non-presentation of operation support information are switched based on a proximity relationship between an insertion unit's distal end and a specific site.
FIG. 21B is a diagram showing, as the third display example, an instance in which presentation and non-presentation of operation support information are switched based on the proximity relationship between the insertion unit's distal end and the specific site.
FIG. 22 is a diagram showing an example of displaying a pickup image and a reconstructed image side by side on a screen.
FIG. 23 is a diagram showing an example of a case where the shape of a large bowel is changed by an inserted insertion unit.
FIG. 24 is a diagram showing examples of the support information or an example of how the support information is generated.

### DETAILED DESCRIPTION

Hereinafter, embodiments of the present invention will be described in detail with reference to the accompanying drawings.

### [First Embodiment]

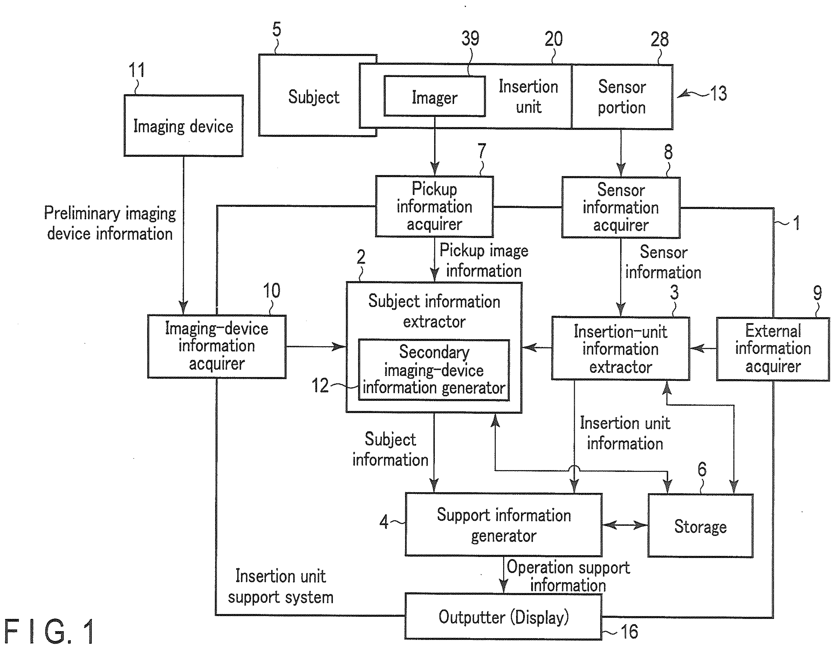

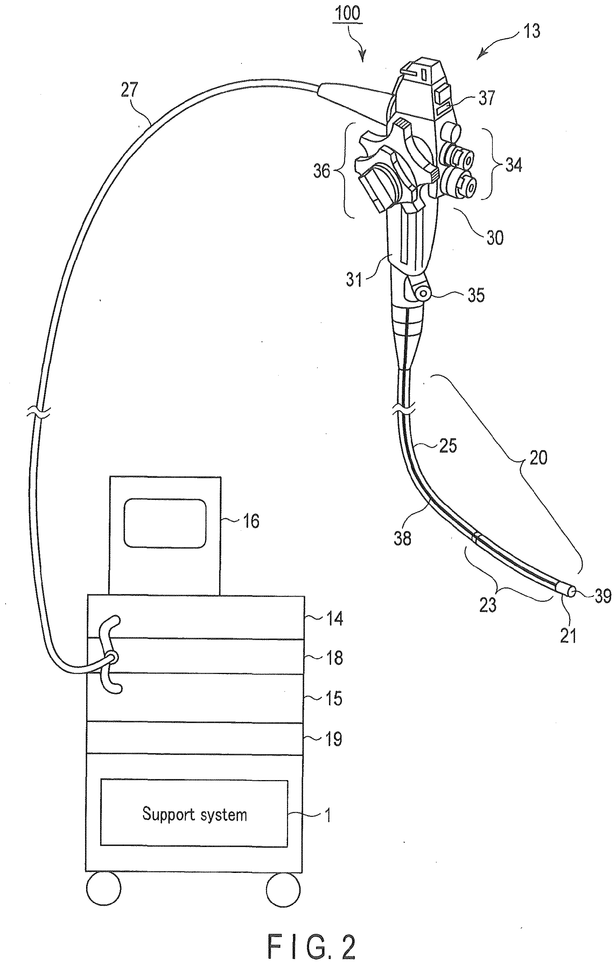

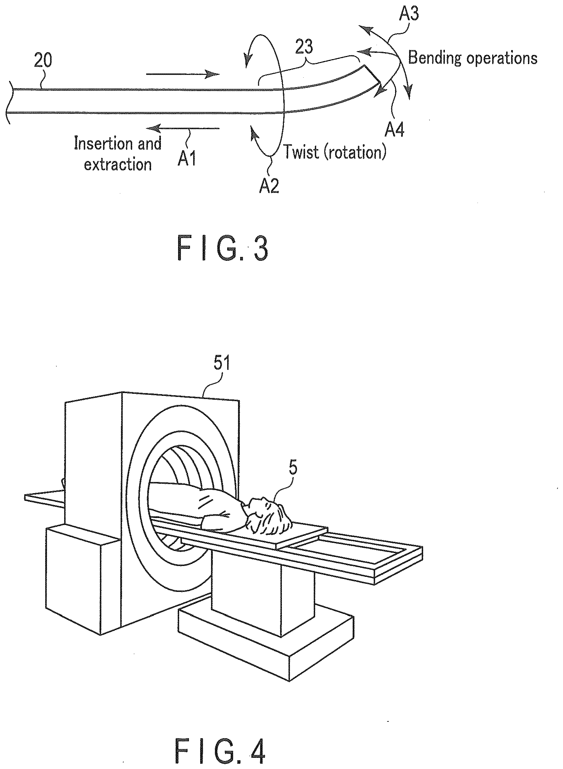

FIG. 1 shows a configuration example of an insertion unit support system provided for a tubular insertion system according to one embodiment of the present invention. FIG. 2 shows external structures of an endoscope having the insertion unit support system, and FIG. 3 shows bending directions of a bending portion at the distal end of an insertion portion of the endoscope. The tubular insertion system according to this embodiment will be described in detail, taking a flexible endoscope for medical use (scope for an upper gastrointestinal tract or a large bowel, scope using ultrasonic waves, cystoscope, pyeloscope, and so on) as an example. As a matter of course, the embodiment is not limited to flexible endoscopes for medical use. The embodiment is broadly applicable to any other tubular insertion systems having a shape bendable in at least a longitudinal portion, such as endoscopes for industrial use, rigid scopes having a partial bending mechanism, manipulators (robot arms), and catheters, as long as they are tubular insertion systems adapted to operate an insertion unit for insertion and treatment.

The following embodiments will assume that the lumen (body cavity or tubular hollow organ) as an observation target of the endoscope refers to a digestive organ, a bronchus, a urinary organ, etc. The description here will use a large bowel as an example. As discussed above, a large bowel is an organ which varies in shape and location for each subject, and which can change its shape over time or according to the insertion of a device, etc. The subject concerned is assumed to be a patient under diagnosis or treatment, but may instead be a patient model or an organ model for simulation. Also, the subject does not have to be in the medical context, but may be devices, etc., having a tubular space or a hollow portion inside.

First, an overall description of an endoscope system 100 that includes an insertion unit support system 1 will be given with reference to FIGS. 2 and 3. FIG. 2 shows an example of the endoscope system's overall structure. FIG. 3 conceptually shows operation-induced movement directions of an insertion unit.

The endoscope system (tubular insertion system) 100 according to this embodiment is a tubular insertion apparatus for insertion into the body cavities of an observation target.

The endoscope system 100 includes an endoscope 13 that acquires images by an imager provided at the distal end of an insertion unit (i.e., insertion portion) for insertion into a subject, an image processor 14 (i.e., video processor) that processes acquired observation images, a monitor (display) 16 that displays the observation images sent from the image processor 14, a light source 18 that provides illumination light for emission to the endoscope 13, a shape sensor (sensor main body) 15, and a controller 19 that takes total control over the endoscope system. It will be supposed that the endoscope according to this embodiment includes generally adopted functions or devices.

The endoscope 13 includes an operation portion 30 having a grip portion 31 for an operator to grip, a long tubular insertion unit 20 connected to the proximal side of the operation portion 30 and having flexibility, and a universal cable 27 connecting the operation portion 30 to both the light source 18 and the image processor 14 and including a signal line for image transmission and a so-called light guide made of an optical fiber for guiding the illumination light. With the operation portion 30, an operator (worker such as a doctor) to operate the endoscope 13 grips the grip portion 31 and uses a bending operation portion 36 and operation buttons 34 arranged above the grip portion 31.

Further, an ID tag 37 is provided above and near the operation portion 30 so that a unique model number, product serial number, etc. for identification are recorded. This ID tag 37 is used when the endoscope 13 is connected to the insertion unit support system 1, for determining supportability or non-supportability and for setting unique parameters. The unique parameters are stored in a later-described storage 6 in advance, and read as appropriate at the time of initialization so as to be set to the controller 19. Note that even when the endoscope is determined to be non-supportable by the insertion unit support system 1, the endoscope may be subject to regular use, without support. Also, when supportability is determined, available support information, and a degree of accuracy or a level of the support information to provide are set as the unique parameters.

Table 1 shows an example of such information written in the ID tag. The ID tag is not necessarily required to have all the information given in Table 1, but would serve the purpose if at least the information necessary for activating the support system and generating support information is written in it.

**[Table 1]**

| Identification Information | Model type/model number, product serial number |
|---|---|
| Constitution and Equipping Options | Insertion portion, bending operation portion, operation portion, shape sensor, ... |
| Specifications | Length of insertion portion, sectional diameters, ..., type of shape sensor, ... |
| Product State and Maintenance History | State: normal, Hours Used: ... hours, Maintenance History: repair of ... , ... |

Based on such information written in the ID tag, the insertion unit support system determines the available support information. The determined available support information may be displayed in, for example, a pull-down menu for an operator to select as needed. This allows the operator to grasp what support information is available and to further set the desired support information to be selectively output.

Moreover, the insertion unit support system performs, as needed, setting for generating available support information or the support information decided to be provided. This includes setting for a sensor or an imaging device to generate and acquire necessary information, arrangement of the steps to receive the information, selection of the processing to generate support information from the received information, and so on. Such setting may be performed upon reaching the decision to generate support information, but should be performed at the time of initialization since this would complete the processing at once at the start, and therefore, the processing can be simplified and troubles in the middle of operations can be avoided.

Further, if a component of the support system is adapted to be attachable and detachable during the activated period, it would be likewise advantageous to have a configuration for detecting the change in system constitution and updating the setting. The insertion unit 20 extends from the proximal side toward the distal side, and is integrally formed so that a flexible tube portion 25, a bending portion 23, and a distal rigid portion 21 are coupled together.

In the insertion unit 20, an optical-fiber light guide (not shown) for guiding at least the illumination light, and a light conducting member, e.g., optical fiber, intended for detection light of the later-described shape sensor 15 for detecting a bent state of the insertion unit 20 are provided side by side and along the longitudinal direction. Note that the optical fiber for the detection light is fixed together with the insertion unit 20 so that the bent state of the insertion unit 20 can be detected. The optical fiber for the detection light is preferably arranged on the central axis of the insertion unit 20 along the longitudinal direction, but its arrangement is not particularly limited. In the following description, the optical fiber for guiding the illumination light is called a light guide, for the sake of distinction. Further, an insertion port 35 for a forceps channel, penetrating through the interior of the insertion unit 20 up to a channel opening at the distal end and allowing various types of forceps to run through it, is provided between the proximal side of the insertion unit 20 and the operation portion 30. Also, an illumination window formed with an optical lens is provided for irradiating the area of an observation target with the illumination light guided through the light guide from the light source 18.

The distal rigid portion 21 is made of a rigid material and formed into a substantially cylindrical shape. While not illustrated, the distal face of the distal rigid portion 21 is provided at least with the illumination window for the emission of the illumination light, an imager 39 including an observation window and an optical system for imaging the area of the observation target, a nozzle for ejecting fluid including a washing liquid and air, and the channel opening. The operation portion 30 is provided with the bending operation portion 36 (with a UD knob, LR knob, etc.) for bending the bending portion 23 in the directions orthogonal to the insertion direction, e.g., up and down directions and left and right directions, and the various operation buttons 34 for performing image acquisition, air and water supply, suction, and so on. The bending portion 23 is constituted by multiple joint rings (not shown), which are coupled in the longitudinal direction of the insertion unit 20 with the rotation axes for connection between the joint rings alternately shifted by 90° to be orthogonal to each other. The distal joint rings are connected to the bending operation portion 36 of the operation portion 30 through multiple wires. The wires are pulled according to the operation on the bending operation portion 36, whereby the joint rings are rotated about the respective rotation axes as appropriate, bringing the joint rings as a whole into a state of forming an arc. As a result, the bending portion is placed into a bent state. Note that the bending operation portion 36 is not limited to manual knobs, but it may be an electricity-driven type that utilizes a driving force of a motor for pulling the wires.

The flexible tube portion 25 has flexibility and can be bent by external force. An operator operates the bending operation portion 36 to bend the bending portion 23 in desired directions, and may further introduce pushing/pulling or twisting behaviors using the flexibility of the flexible tube portion 25. Thereby, discretional insertion into the digestive organ, bronchus, urinary organ, etc. of a subject patient can be done. Moreover, bending the bending portion 23 changes the orientation of the distal rigid portion 21 so that the observation target falls within the observation view field and is irradiated with the illumination light.

According to this embodiment, the shape sensor 15 arranges at least one detection subject portion 38 for detecting a bent state, at predetermined positions of the optical fiber disposed inside the insertion unit 20. The shape sensor 15 includes a light source, which will be described later, to emit detection light that differs in wavelength from the illumination light through the universal cable 27 (optical fiber). The shape sensor 15 emits the detection light within the optical fiber in the insertion unit 20, and detects the detection light influenced by the detection subject portion 38 so that the shape of the insertion unit 20 at that time is detected.

In addition to the shape sensor 15, the endoscope 13 further includes an insertion unit sensor 86 and an operational amount sensor, which will be described later, to constitute a sensor portion 28. The operational amount sensor is disposed in the operation portion 30 described above, and adapted to detect the movement amounts of the multiple wires connected to the bending operation portion 36. These movement amounts of the wires are changed according to the operational amount of the bending operation portion 36, and thus, the movement amounts of the wires enable detection of the bending direction and bent state of the bending portion 23.

The sensor portion 28 detects/estimates at least one of arrangement information including the shape and orientation of the insertion unit 20, and information (position, direction, and orientation) about the distal end, so that operation support information can be generated from insertion unit information based on sensor information obtained by the sensor portion 28 and subject information based on information of the imaging device. At least one of the arrangement of the insertion unit 20 including the shape and the orientation, and the information (position, direction, and orientation) about the distal end forms part of the insertion unit information and the operation support information. Accordingly, an operator can know position-associated information of the insertion unit 20, and understand how deep the insertion unit 20 has been inserted into a lumen or how the insertion unit 20 should proceed with further insertion, more accurately than in the cases of operations without this information. Thus, the combination with the subject information will improve the insertion unit's insertability and operability.

Using these sensors, the sensor information is generated for a forward/backward movement amount A1 in the direction of insertion/extraction of the insertion unit 20 inside a lumen, a twist (rotation) amount A2 accompanying the rotation of the operation portion 30, and the bending directions, i.e., up/down direction A3 and left/right direction A4, and movement amount of the bending portion 23 caused by the operation on the bending operation portion 36, as shown by arrows in FIG. 3.

Note that the endoscope 13 may include sensors other than these sensors, and also actuators. The sensor information may be detected or generated by one or more sensors included, and the obtained sensor information may consist of multiple types of information.

Referring to FIG. 1, the configuration of the insertion unit support system 1 will be described.

The insertion unit support system 1 according to this embodiment is provided for the endoscope system 100. It provides support information for doctors as operators during insertion and extraction operations with a patient as a subject 5, and supports their observation and treatment for diagnosis and medical remedy. More specifically, at the time of insertion and extraction operations or treatment operations of the endoscope 13, the insertion unit support system 1 generates the operation support information by taking in image information obtained at the endoscope's imager, and by importing the sensor information detected by each sensor described later, and preliminary image-associated information such as images acquired by imaging devices, information generated based on the images, and an assessment added to the information. The insertion unit support system 1 then provides the operation support information to an operator.

The insertion unit support system 1 primarily includes a subject information extractor 2, an insertion-unit information extractor 3, a support information generator 4, a storage 6, an outputter (e.g., display) 16, and multiple information acquirers described later. The insertion unit support system's outputter 16 serves also as the display of the endoscope system 100. The outputter 16 is not limited to the function of outputting image information (operation support information) on the display, but may include a function of outputting various types of information toward other devices, etc. As a matter of course, the functions may be realized by discrete outputters.

The multiple information acquirers externally acquire various types of subject information about the subject 5 as an observation target. The information acquirers in this embodiment include a pickup information acquirer 7, a sensor information acquirer 8, an external information acquirer 9, and an imaging-device information acquirer 10. As a matter of course, these information acquirers may be incorporated as part of the subject information extractor 2 or the insertion-unit information extractor 3. Also, in addition to these information acquirers, other members capable of providing available information may be included.

More specifically, the pickup information acquirer 7 takes in pickup image information including the image of the subject 5 taken by the imager 39 at the distal end of the insertion unit 20, and sends it to the subject information extractor 2. The pickup image information is image information about the lumen of the subject 5.

The sensor information acquirer 8 acquires sensor information including insertion unit information detected by the shape sensor 15, as well as insertion unit information detected by the later-described insertion unit sensor 86 and operational amount sensor, and sends the sensor information to the insertion-unit information extractor 3.

Further, the external information acquirer 9 acquires external information about the subject 5 obtained via external devices or a LAN before endoscope observation or endoscope diagnosis, and sends it to the insertion-unit information extractor 3. The imaging-device information acquirer 10 acquires imaging device information such as three-dimensional tomograms of the subject 5 from a later-described imaging device 11 before endoscope observation, and sends it to the subject information extractor 2. Each information acquirer may also obtain information, etc. for operation support, in addition to the subject information and the insertion unit information.

Moreover, the external information acquired by the pickup information acquirer 7, the sensor information acquirer 8, the external information acquirer 9, the imaging-device information acquirer 10, etc. is subjected to appropriate extraction processing, modification processing, etc., within the insertion unit support system 1 or using an external device at the request of the insertion unit support system 1. The information imported from the outside by the insertion unit support system 1 will be assumed to be primary information, and the information generated within the insertion unit support system 1 or generated based on the instruction of the insertion unit support system 1 will be called secondary information. Also, the primary information and the secondary information may be collectively and simply called information. For example, information obtained beforehand on the imaging device 11 will be called primary information, even if it has been preprocessed for use by the insertion unit support system 1. On the other hand, when the insertion unit support system 1 instructs the imaging device 11 that has provided imaging device information to generate specific information afresh based on the already imported information about the subject 5, the information obtained in such an instance will be called secondary information.

Among these information acquirers, the imaging-device information acquirer 10 and the imaging device 11 are essential. For the others, it is sufficient if there are at least one or more devices and information from these devices and the information from the imaging-device information acquirer 10 are combined so that the insertion-unit information extractor 3 can extract or generate the insertion unit information.

The subject information extractor 2 extracts or generates the subject information such as the shape of the subject 5 from the acquired sensor information and imaging device information. The insertion-unit information extractor 3 extracts or generates the insertion unit information indicative of the insertion state, form, etc. of the subject 5 from the acquired sensor information and external information. The support information generator 4 generates the operation support information as will be described, by sorting the insertion unit information from the subject information extractor 2 and the insertion-unit information extractor 3 and adding various processing to it.

The storage 6 stores information about subjects and operators, information obtained from the multiple information acquirers, intermediate information or support information in the subject information extractor 2, the insertion-unit information extractor 3, and the support information generator 4, unique parameters of various endoscopes and sensors, and so on. More than one storage 6 may be provided for respective purposes, or the storage 6 may be provided externally and connected via a LAN, the Internet, etc. As discussed above, the outputter 16 includes a display for displaying the operation support information from the support information generator 4, as one form of output. The display of the support information is a form of output, and there are a variety of other available output forms. For example, presenting information may take the form of sound, vibration, light, and other various forms. The operation support information undergoes conversion or changes in accordance with the output form. Operations such as controlling an actuator based on the support information are also possible.

Each component of the insertion unit support system 1 will be described in detail.

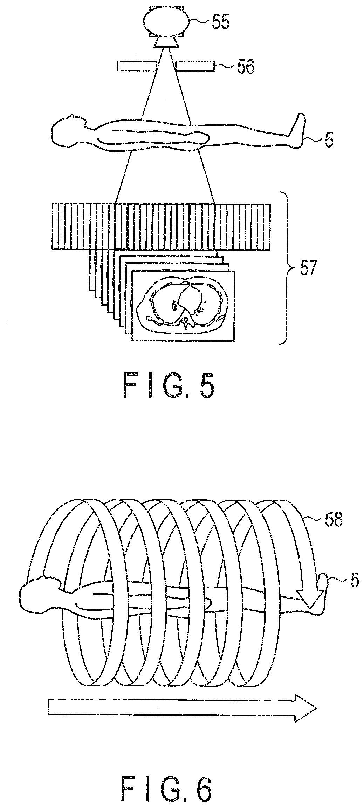

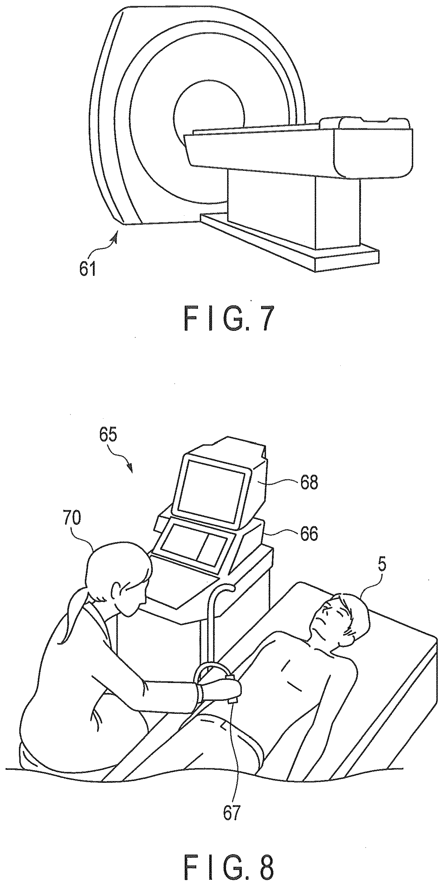

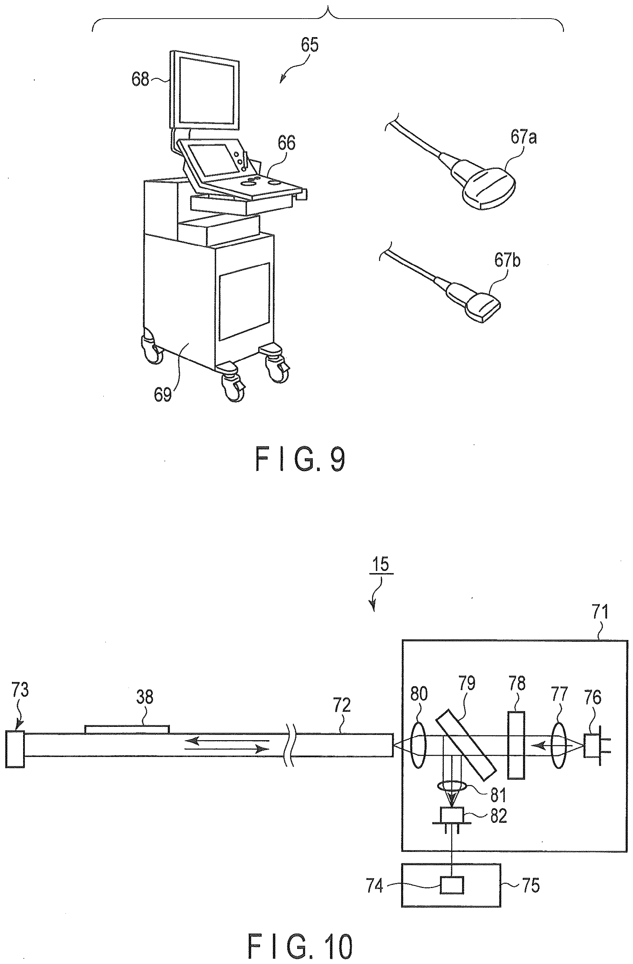

FIG. 4 shows external structures of a CT apparatus, FIG. 5 conceptually shows images of a subject taken from a specific viewpoint, and FIG. 6 shows a concept of helical-scan imaging in a CT apparatus. FIG. 7 is a diagram showing external structures of an MRI apparatus. FIG. 8 shows a diagnostic situation using an ultrasonic diagnosis apparatus, and FIG. 9 shows external structures of an ultrasound diagnostic apparatus and probes for use.

The imaging device 11 used in this embodiment may be a known device. For example, devices such as a CT apparatus 51, an MRI apparatus 61, and an ultrasonic diagnosis apparatus 65 may be adopted. From such imaging devices 11, two- or higher dimensional image information about the lumen of the subject 5 as an observation target is acquired just before endoscope observation. Or, image information used for other purposes, e.g., diagnosis, just before the endoscope observation may be re-used. As the image information applicable to this embodiment, the information shown in below Table 2 is available. Depending on the imaging device 11, some information may not be available, and such information will be excluded.

**[Table 2]**

| Imaging Device Information | Explanation/Concrete Example |
|---|---|
| Image Data | Raw image data, processed image data (images from a specific direction/specific viewpoint, or three-dimensional images) |
| Specific Site Data | Images (extracted images) of specific site, etc., length, area, volume, and shape of the site, ... |
| | Specific site: Picked up site of lumen, involving a narrowed portion, portion suspected of a polyp, etc., and portion associated with insertion or operation of the insertion unit |
| Examination and Diagnosis Data | Results of examination and diagnosis for subjects/diseased parts by apparatuses/engineers/doctors using the imaging device, including disease names/wound types, name of lumen site as a diseased part, degree, stage, etc. of a lesion or wound/damage or malfunction |

In particular, the CT apparatus 51 is capable of observing the entire range of an observation target via a collimater 56, in the specific direction from the outside with respect to the subject 5 or from the viewpoint of an imager 55 as shown in FIG. 5, and can acquire image information 57 covering a broad range or the whole of the subject 5 from one direction or one viewpoint. Also, as shown in FIGS. 5 and 6, helical scanning 58 that moves the viewpoint (subject) during an imaging operation enables the reconstruction of a stereoscopic image or any given sectional shape.

The ultrasonic diagnosis apparatus will be explained. In endoscope observation, as discussed above, it is most preferable that the insertion unit 20 for observation is inserted into the subject 5, and while this inserted state is kept, information for the ongoing situation is acquired sequentially.

As shown in FIGS. 8 and 9, the ultrasonic diagnosis apparatus 65 includes an apparatus body 66, a probe 67 (67a, 67b), and a monitor 68, and is placed on a caster rack 69 for movement. The apparatus body 66 includes, while not illustrated, a transmitter/receiver for generating and transmitting/receiving ultrasonic waves, a controller, a signal processor for processing Doppler signals, etc., an image processor, a recorder, an inputter such as a keyboard, and so on. As a typical probe, the probe 67a of a convex type for radially emitting ultrasonic waves, and the probe 67b of a linear type for linearly emitting ultrasonic waves are shown. Other than these, a sector-type probe could be used for an area narrower than the convex-type probe 67a. To use the ultrasonic diagnosis apparatus 65, the subject 5 is made to lie on an examination table as shown in FIG. 8, and a doctor 70 places the probe 67 on the part intended for observation and acquires three-dimensional images.

Note that in usual endoscope observations, a patient as the subject 5 is often laid down. Accordingly, the patient adopts a posture similar to the case of observing internal images using the imaging device 11. Due to the different posture of the lying body, an organ can vary in location to some extent due to gravity. Thus, if possible, the imaging device information is adjusted as needed to indicate an image that accords with the endoscope observation.

Also, when a subject is an article, a device, etc., an imaging device suitable for the subject will be used. For example, depending on the material of the subject, an infrared imaging device may be used to obtain perspective images.

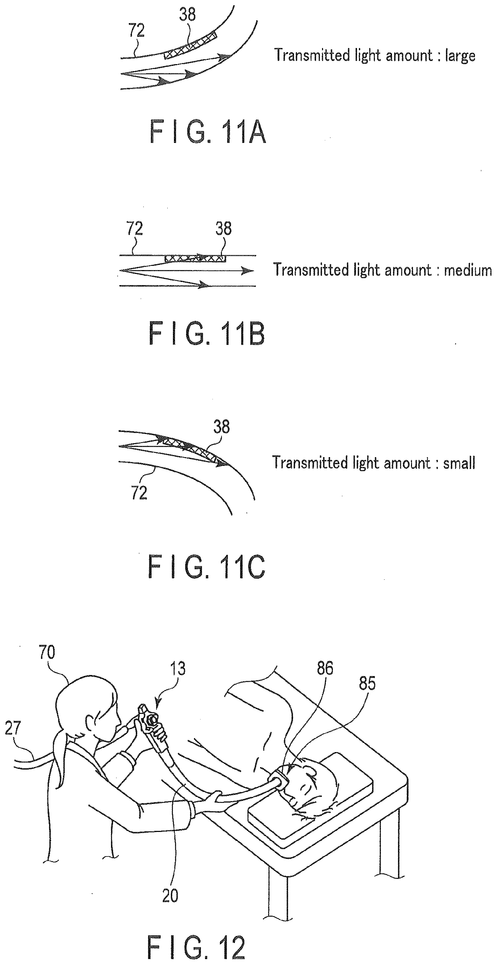

Next, the sensor information acquired by the sensor information acquirer 8 will be described with reference to FIGS. 10 to 12. FIG. 10 shows a configuration example of the shape sensor 15 for detecting the bending form of the insertion unit 20. FIGS. 11A, 11B, and 11C are conceptual diagrams for explaining the amount of transmitted light at the time of bending operations, in relation to a fiber sensor attached to an optical fiber. FIG. 12 shows an example of setting the insertion unit sensor 86 at an opening (e.g., oral cavity) 85 of the lumen of the subject 5.

The sensor information in this embodiment includes a shape, an inserted distance (insertion amount), etc. of the insertion unit 20, obtained by processing the information detected by the shape sensor 15, insertion unit sensor 86, and the operational amount sensor.

The shape sensor 15 shown in FIG. 10 arranges at least one detection subject portion 38 in an optical fiber 72 disposed along the longitudinal direction of the insertion unit 20 of the endoscope 13 as shown in FIG. 2, and obtains the bending form of the insertion unit 20 from a detected curvature (bending amount).

The shape sensor 15 includes a sensor main body 71, the detection subject portion 38, and a controller 75. The controller 75 includes a shape calculator 74 and performs integrated control of the components within the sensor main body 71. As described, the optical fiber 72 and the light guide (optical fiber) for guiding illumination light are disposed side by side.

The sensor main body 71 includes a light source 76, a projector lens 77, an isolator 78, a reflective mirror 79, and a first condenser lens 80 arranged in this order on the optical axis from the emitting side. A second condenser lens 81 and an optical detector 82 are arranged on the optical axis that has diverged at the reflective mirror 79.

The light source 76 is formed of, for example, an LED and emits detection light having at least one wavelength differing from the illumination light used in the endoscope. When there is more than one detection subject portion 38, a configuration to use detection light having a plurality of different wavelengths is preferable. The isolator 78 allows the detection light emitted from the light source 76 to pass through, while prohibiting the passage of the detection light reflected and returned from the reflective mirror 79, thereby preventing the light from returning to the light source 76.

The detection light having exited from the condenser lens 80 of the sensor main body 71 enters the optical fiber 72 from the proximal side and is guided. The guided detection light is reflected at a reflector 73 provided at the distal end of the optical fiber 72, and transmitted within the optical fiber 72 again to return to the sensor main body 71 for detection. This detection light is refracted and diverged at the reflective mirror 79 and received by the optical detector 82. The optical detector 82 includes photoelectric conversion elements, etc. and outputs shape signals based on the optical intensity of the detection light that varies due to bending operations. The shape calculator 74 calculates and outputs the actual curvature (degree of arc) of the bending form of the bending portion 23 based on the shape signal from the optical detector 82.

The relationship between the curvature of the insertion unit 20 and the transmitted light amount of the detection light will be described.

In FIG. 11A, arrows show the detection light guided within the optical fiber 72 bent in the direction of the side where the detection subject portion 38 is attached. In FIG. 11B, arrows show the detection light guided within the optical fiber 72 in a straightened state. In FIG. 11C, arrows show the detection light guided within the optical fiber 72 bent in the direction opposite to the side where the detection subject portion 38 is attached.

As described above, the detection subject portion 38 is attached at the periphery of the specific portion of the optical fiber 72 and absorbs the guided detection light to reduce its optical intensity, that is, the transmitted light amount. Accordingly, the greater the light amount emitted to the detection subject portion 38, the smaller the transmitted light amount. In other words, when the optical fiber 72 is changed from the straightened state shown in FIG. 11B to the bent state shown in either FIG. 11A or 11C, the transmitted amount of the detection light will also decrease or increase in synchronism. This change in the transmitted light amount of the detection light produces a change in the intensity of light received by the optical detector 82, and the shape calculator 74 calculates the curvature of the optical fiber 72 from the bending direction at the detection subject portion 38 and the detection signal based on the change in the light amount. In these examples, the bent state shown in FIG. 11A, in which a large portion of the light is reflected by the side wall of the optical fiber, transmits the largest light amount. Then, the amount of the transmitted light decreases in the order of the straightened state shown in FIG. 11B, in which a portion of the light is incident in the detection subject portion 38, and the bent state shown in FIG. 11C, in which a large portion of the light is incident in the detection subject portion 38.

As such, the shape sensor 15 is a fiber sensor of a type that detects change in light amount of the light traveling within the optical fiber 72, due to the bending form of the optical fiber 72. As the characteristics thereof, it is thin and can easily be incorporated into endoscopes, and it is mostly unaffected by other structures. This type of sensor enables formation of detection parts at low cost, and therefore contributes to mass-produced products. Other than this type, an FBG type, in which an optical fiber is formed with a grating, could also be used. According to this type, while the detection parts might be complicated and expensive, this would allow for highly accurate detection of a bending form within a desired range by providing multiple detection subject portions 38 at one optical fiber and thus realizing multiple detection points.

FIG. 12 illustrates an example of the configuration of the insertion unit sensor 86. The insertion unit sensor 86 optically reads a position-indicating mark (not shown) at the outer periphery of the insertion unit 20 by an optical sensor, etc., and detects the insertion amount and the rotation amount of the insertion unit 20 with respect to a body cavity (lumen).

The sensor portion 28 adopted in this embodiment is constituted by the shape sensor 15, the insertion unit sensor 86, and the operational amount sensor, and the following sensor information can be obtained by having these sensors.
1) Insertion unit's bending form
2) Insertion unit's insertion amount
3) Rotation (twist) amount
4) Operational amount of bending the bending portion at the distal end of the insertion unit
5) Insertion unit's bending form with respect to a subject having a lumen
6) Force applied to the distal end of the insertion unit

Among these, the sensor information 2) to 4) are obtained directly from the respective sensors, and the sensor information 1), 5) and 6) are obtained by signal processing. More specifically, as to the sensor information 1), bending information by the shape sensor may be combined so that the bending form in a given range can be calculated. The sensor information 5) can be calculated using the sensor information 1) to 3). The sensor information 6) can be calculated using the sensor information 1) and 4).

Also, the sensors for use are not particularly limited to the shape sensor, the insertion-unit sensor, or the operational amount sensor, but any type of sensor may be adopted as long as equivalent information can be acquired. As a matter of course, a number of same-type sensors may be arranged for acquiring the sensor information. Moreover, the information is not limited to the contents discussed above, but different types of sensors may be adopted for acquiring different types of information, as long as the information can be utilized in the insertion unit support system 1.

Next, the external information acquired by the external information acquirer 9 will be described.

As the external information acquired from external devices via the external information acquirer 9, external information shown in Table 3 may be named. Such external information is not an absolute requisite in the insertion unit support system 1 according to this embodiment, but it is often useful. Thus, the information may be selected and used according to purposes. Among the external information, the information that can be obtained beforehand may be stored in the storage 6, updated as appropriate, and then read out as needed.

**[Table 3]**

| | |
|---|---|
| Subject Information Obtained via External Devices or LAN | Vital information, motion, etc. of a subject (information, such as positional relationship between a subject and the insertion unit, for obtaining insertion unit-associated information is included in the sensor information, and excluded from the external information) |
| Examination and Diagnosis Data | Results of examination and diagnosis for subjects/diseased parts by apparatuses/engineers/doctors, including disease names/wound types, name of lumen site as a diseased part, degree, stage, etc. of a lesion or wound/damage or malfunction (information acquired from the imaging devices is excluded) |
| Information Associated with Generation of Support Information | Information that can be referred to when generating the operation support information, including patient's past medical records, treatment method for a lesion part, specification of an endoscope, etc. |

Next, the subject information extractor 2 and the insertion-unit information extractor 3 will be described.

For endoscope observation, the subject information extractor 2 extracts or generates subject information that may mainly include the imaging device information (preliminary imaging device information) acquired from the imaging device 11 beforehand in relation to the subject 5 and that is necessary for generating the operation support information. The information obtained from the external information acquirer 9 may be used together for extracting or generating operations. In the below descriptions, the secondary information generated by a secondary imaging-device information generator 12 based on the imaging device information from the imaging device 11 will be called "secondary imaging-device information".

It is not always the case that the imaging device information is directly usable for operation support. In particular, image information from a specific viewpoint, lesion information, etc., is useful but may not be included in the imaging device information. In that case, such information is generated by the secondary imaging-device information generator as secondary imaging-device information so that desired operation support information can be generated.

The secondary imaging-device information includes the following. Note that information that has already been acquired as imaging device information will be excluded.
1) Secondary image information: An image which, when the tubular insertion unit 20 includes an imager, is reconstructed to correspond to the image of an imaging site taken by the imager (reconstructed by designating the position and orientation of the imaging site for the imaging device 11).
2) Secondary shape and location information
3) Secondary specific-site information

This secondary imaging-device information includes secondary lumen shape and location information indicative of the shape or the location of a lumen based on a specific viewpoint/cross-section. The secondary imaging-device information generator 12 may receive a designation of to-be-generated information from the insertion-unit information extractor 3 and generate the secondary information based on the designation. For example, it is possible to refer to a pickup image taken by the imager 39 and the position and orientation of the insertion unit acquired by each sensor so that an image corresponding to the pickup image is reconstructed from the imaging device information. Also, it is not a requisite that the generation of the secondary imaging-device information is performed in the subject information extractor 2, but it may be outsourced to external devices including the imaging device 11.

Since the secondary imaging-device information includes the secondary lumen shape and location information, the insertion unit's insertability and operability is improved by matching the specific viewpoint of the secondary image information for image reconstruction with the viewpoint of an operator or the viewpoint of the insertion unit's imager. Also, by setting a viewpoint that would facilitate the recognition of conditions of a specific site, the manner to pass through the specific site or the manner to arrange the insertion unit for suitable operations at the specific site, such as observation/diagnosis or treatment, will be further improved, contributing to the insertion unit's insertability and operability.

The shape and the location of a lumen as an observation target is not directly observable from the outside of the subject, but can be precisely grasped if the secondary lumen shape and location information is available, as compared to the cases where information about the movement direction or the entrance position of the insertion unit is absent.

In addition, unlike in the cases where the secondary specific-site information is absent in the secondary imaging-device information, it is possible to prevent a specific site from being overlooked, and therefore, the time required for search can be reduced. Moreover, even when an operator is already aware of the secondary specific-site information, the operator may receive it as needed in the form of the operation support information. Such setting can reduce the possibility of a site being overlooked, especially when the number and types of the specific sites are many, contributing to the improvement of the insertion unit's insertability and operability.

The secondary imaging-device information corresponding to a pickup image is generated from the shape and location information or distal end information of the insertion unit 20, and the imaging device information. Also, in the insertion unit support system 1, the secondary imaging-device information includes the secondary lumen shape and location information indicative of the shape and the location of a lumen based on a specific viewpoint/cross-section, the insertion unit information includes the shape and arrangement information of the insertion unit based on a specific viewpoint/cross-section, and the operation support information includes image information combining the secondary lumen shape and location information and the insertion unit's shape and arrangement information. The operation support information, as it contains the image information combining the secondary lumen shape and location information and the insertion unit's shape and arrangement information, allows for the instant confirmation of the positional relationship of the insertion unit relative to lumens, and therefore, the insertion unit's insertability and operability are improved.

The subject information and the operation support information discussed above include the secondary specific-site information corresponding to a specific site that requires attention in the insertion and extraction of the insertion unit 20, that is, a site that must be carefully considered during the insertion and extraction or a site targeted for operations such as observation/diagnosis or treatment. As such, the display of the range/shape/size of a specific site and the information including the contents/operations/caution points are presented as the operation support information together with the image of a lumen corresponding to an endoscope image, and the identification of the specific site and operations on the endoscope's pickup image are thereby facilitated, improving the insertion unit's insertability and operability.

The insertion-unit information extractor 3 extracts or generates the insertion unit information necessary for generating the operation support information, using the sensor information relevant to the insertion unit 20 and input from the sensor information acquirer 8. This sensor information is, as described above, information detected from the shape sensor 15, the insertion unit sensor 86, and the operational amount sensor. Further, the external information relevant to the insertion unit 20 and obtained from the external information acquirer 9 may be used, or combined with the sensor information, for extracting or generating operations.

Even after an observation region or a target region for treatment operations, etc., is confirmed using the insertion unit 20, the region could shift its location, disappear, or appear in other sites, or a lesion could be found different than the expected type. In such cases, the insertion-unit information extractor 3 corrects the subject information or updates it on a real-time basis based on the imaging device information (pickup image information) or at least one of the sensor information. This allows for the acquisition of lumen information different from the imaging device information as preliminary information, at the time of inserting the insertion unit 20 into a lumen. Also, this enables the acquired lumen information to change moment to moment according to the insertion and operation situation. Performing such real-time correction/update of the subject information will assist in the next insertion of the insertion unit, diagnosis, or treatment.

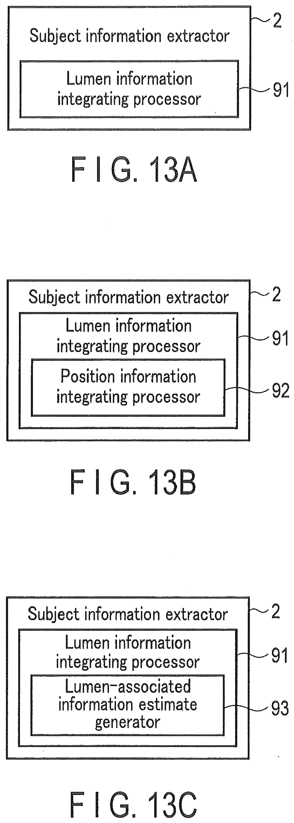

Referring to FIGS. 13A to 13D, the subject information extractor 2 will be described.

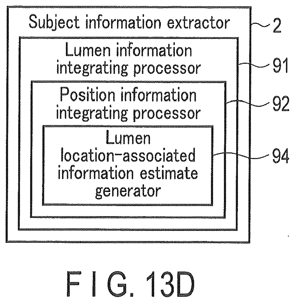

FIG. 13A shows a lumen information integrating processor 91 provided in the subject information extractor 2. FIG. 13B shows a position information integrating processor 92 provided further in the lumen information integrating processor 91. FIG. 13C shows a lumen-associated information estimate generator 93 provided also in the lumen information integrating processor 91. FIG. 13D shows a lumen location-associated information estimate generator 94 provided in the position information integrating processor 92.

As shown in FIG. 13A, the subject information extractor 2 includes the lumen information integrating processor 91 for integrating information about a lumen of the subject 5, contained in the imaging device information and the sensor information. This lumen information integrating processor may be provided in parallel with the subject information extractor 2. Information to be integrated is not particularly limited, as long as it concerns a lumen. For example, when one condition is expressed differently, or when redundant information, omission of information, or inconsistency between a condition and expression is involved, such information, etc. are integrated in accordance with a predetermined style so that the information becomes concise and easy to handle.

Moreover, examples of the information to be integrated include, in particular, position-associated information related to the position of a specific site. As the position-associated information, the shape, size, position, orientation, etc. of a specific site may be cited. The position information integrating processor 92 shown in FIG. 13B, provided in the lumen information integrating processor 91, applies a common coordinate system to these types of position-associated information in order to integrate the position information. By integrating the coordinate systems of the position-associated information into one, it is possible to process or display the position information from both sides using the common coordinate system.

On the other hand, when such integration of coordinate systems is performed, it is often the case that position information falling within the interval of arrangement required for generating or presenting the support information will be lost, or the position information will be insufficient for generation of the support information without undergoing particular processing. To address such instances, the lumen-associated information estimate generator 93 is provided in the lumen information integrating processor 91 as shown in FIG. 13C so that position information required for generating or presenting the support information is additionally generated through interpolation or estimation.

When the lumen information included in the preliminary information from the imaging device 11 and that in the sensor information involve an inconsistency or a deficiency, for example, when different shapes, sizes, positions, orientations, etc., of the specific site are shown, or when information required for generating the support information is missing, this lumen-associated information estimate generator 93 estimates the current lumen information from multiple sets of information or estimates the necessary but missing information as much as possible, so that the information is additionally generated and the lumen information is integrated. Also, for generating information through the estimation about positions, the lumen location-associated information estimate generator 94 is provided in the position information integrating processor 92 of FIG. 13B, as shown in FIG. 13D, so as to estimate the position-associated information.

Note that, contrary to the imaging device information as preliminary information acquired from the imaging device 11, the sensor information is the latest information. If it is found that necessary information is missing in view of the current lumen information estimated based on the assumption that this latest information is accurate, estimation is performed for covering the missing information as much as possible, and the information is added at the time of integration. Thereby, the support information can be generated or presented with a higher accuracy.

Now, the integration of information will be described, taking the combination of the CT apparatus 51 and the endoscope 13 as an example. Specifically, assuming that the image information (three-dimensional tomograms, etc.) acquired from the CT apparatus 51 and the sensor information will be integrated, descriptions will be given of how to obtain coordinate systems in the imaging device 11 and the insertion unit support system 1, how to determine the direction of a subject, and so on.

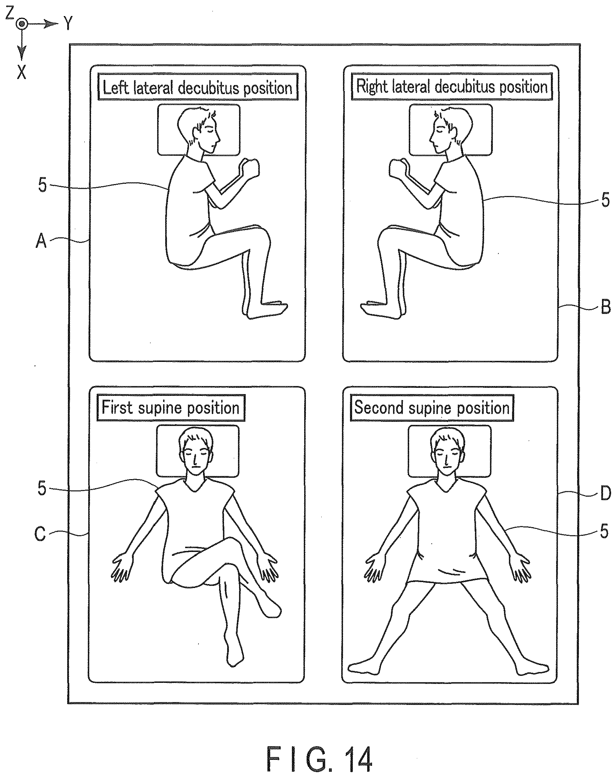

FIG. 14 shows four lying states (left lateral decubitus position A, right lateral decubitus position B, supine position C, and supine position D) of the subject 5 placed on an examination table. Relating to this instance, the examination table used with the CT apparatus 51 will be called an examination table M, and the examination table used with the endoscope 13 will be called an examination table N.

First, two coordinate systems are set in a manner that enables integration.

Where the subject 5 lies on the examination table M, the direction of each coordinate axis of the XYZ-coordinate for the subject 5 is defined as follows. The XYZ-coordinate for the subject 5 is set with X: longitudinal direction of the examination table, Y: lateral direction of the examination table, and Z: above the examination table, as shown in FIG. 14.

As a method to set a coordinate system that suits the subject 5 more accurately than this coordinate setting, the following methods may be selected and adopted as appropriate.
1) Set the X and Y directions based on an orientation of the backbone (approximately in an abdominal area), pelvis, etc.
2) Set the X and Y directions based on a line connecting the head and the groin/foot end.
3) Put a number of markers that will appear in CT images on the subject 5, take CT images, and set the X and Y directions based on the positions of the markers.
4) Discretionarily determine the X and Y directions by an operator, e.g., doctor, and set them through inputting.

Likewise, the Z direction is also determined from CT image information.

Next, the XY-coordinate system is set in the insertion unit support system 1. Examples of this setting may include the following methods.
1) Provide a marking, e.g., a line extending in the X/Y direction, on the examination table N, and adjust the orientation of the body of the subject 5 to match the markers of the subject 5, which have been put at the time of the operation on the examination table M, with the marking.
2) Fix the subject 5 using a tool that presses or holds the subject 5 from the back or both sides of the abdominal part. In addition, the coordinate system for a sensing system is matched with the coordinate system of the examination table N.

If changes of the subject's posture while the subject is on the examination table N are expected, the coordinate system for the subject may be directly set by putting markers on the subject, by taking images of the subject using a camera and performing image processing, or the like. How to obtain the coordinate systems in this embodiment is only an example, and any coordinate system, including a coordinate system having a different coordinate origin or different directions, polar coordinate system, etc., may be adopted as long as the coordinate system allows for unique identification.

Subsequently, the extraction of the lumen shape and location information of the subject 5 from image information of CT, etc., and the coordinate system conversion are performed in the following manner.
1) Extract the shape and location information about the lumen (organ, etc.) of the subject 5, into which the insertion unit 20 will be inserted.
2) Allow the shape and location of the lumen of the subject 5 to be rendered on the coordinate system of the sensor. Also, the reconstruction of the lumen image from a specific direction (viewpoint) is performed as follows.
3) Send the direction/viewpoint information on the CT information coordinate system from the insertion unit support system 1 to a CT apparatus that generated CT information, and cause the CT apparatus to perform reconstruction. Further, CT information raw data and the direction/viewpoint information on the CT information coordinate system are sent to other devices or within the insertion unit support system 1 for reconstruction.

Thereafter, the extraction of necessary information about a specific site, such as a lesion or treatment portion, and the coordinate conversion are performed in the following manner.
1) Extract a distorted structural portion in the shape information about the organ of the subject 5 through pattern extraction or operator's visual recognition.
2) Extract a portion in the CT information containing an organ or tissue adjacent to the organ that involves a color or pattern information corresponding to a particular tissue (cancer, etc.).

Regarding the handling of the instances where the lumen information varies between the time of CT imaging and the time of inserting the insertion unit 20, the following a) to e) show the contents of the variations and how to deal with them.
a) For the variation in the position/orientation of the distal end of the insertion unit 20 with respect to the lumen (organ) of the subject 5, reconfigure the insertion unit's insertion route into the lumen. For this reconfiguration, the following can be considered for reference: to make the shortest connection to the route of a minimum changing rate of the lumen shape /the route of a constant lumen (organ) length /the conventional lumen route.
b) For the variation in size of the specific site of the subject 5, which is due to a gas injection/aspiration or insertion operation, injuries or diseases, etc., change the shape of the specific site or the surrounding area thereof in accordance with the increase-decrease rate of the lumen's cross-section area.
b-1) This variation correction is performed so that the wall size will increase if the lumen's cross-section area decreases, and contrarily, the wall size will decrease if the lumen's cross-section area increases.
b-2) As a first length change, which covers the length of the lumen up to the point the insertion unit 20 has been inserted, the lumen would be changed to take the shape and location according to the insertion unit 20, and to extend or contract depending on the portion of the lumen near the distal end of the insertion unit 20 at the inserted point and on the change in insertion amount (length of insertion) of the insertion unit.
b-3) As a second length change, which concerns the length of the portion beyond the inserted point of the insertion unit 20, no correction needs to be made, as one option.
   Moreover, as another method, changes of the shape and location if further insertion is made are predicted in relation to, in particular, the extension and contraction of the large bowel.
c) Introduction of new items about the lumen (organ) of the subject 5
   Once new items such as a lesion, treatment trace, adhesion, etc. are identified at the time of insertion of the insertion unit 20, such items can be added to the lumen information.
   The methods for addition may include the following two methods.
c1) Automatically extract and add.
c2) Confirm and assess by an operator, and extract and additionally input.
d) Biased locations of the internal parts (organs) of the subject 5 due to the difference in lying state of the subject 5
   If, during the image information acquisition and the insertion of the insertion unit, there are changes due to the lying posture of the subject 5 such as the left lateral decubitus position, right lateral decubitus position, first supine position, second supine position, etc., as shown in FIG. 14, the movement of the particular lumen and the surrounding components (organs, etc.) is estimated from the CT image information based on the change in lying state.
e) Occurrence of shape or location variations due to the change in body shape/shape of the specific site, as a result of time passage

If there are changes such as an aging variation from the time of image information acquisition, changes in shape and location of the specific site or the whole subject are estimated from information such as a body weight, abdominal circumference, body fat percentage, amount of visceral fat, etc.

Next, the support information generator 4 will be described.

The support information generator 4 generates the operation support information by sorting and taking in the subject information output from the subject information extractor 2 and the insertion unit information output from the insertion-unit information extractor 3 which are described above, or by processing the information as appropriate. The operation support information includes a combination of the subject information and the insertion unit information, the subject/insertion unit information derived from the combination of the subject information and the insertion unit information, information relevant to the insertion unit's operations (recommending operations, instructions, and warning), etc. These types of operation support information are shown in Table 4 below.

**[Table 4]**

| Support Information | Concrete Examples |
|---|---|
| Images | 2D and 3D images of lumen based on the imaging device information |
| | Specific site images based on the imaging device information, in particular, images having a substantially consistent viewpoint or size corresponding to the pickup image |
| Location and Shape Data | Location and shape (2D or 3D) of lumen, based on the imaging device information |
| | Arrangement and shape (including the position and orientation of the distal end, insertion amount, etc.) of insertion unit, based on the sensor information |
| | Simultaneous display of these |
| Specific Site Data | Images (extracted images) and positions of the specific site, etc. |
| | Length, area, volume, shape, ... of the specific site, etc. |
| | Currently reached site, and distance, expected insertion period, etc., up to the target site |
| | Characteristics, diagnosis data, etc., about the currently reached site |
| Diagnosis Data | Results of diagnosis for subjects/diseased parts by apparatuses/engineers/doctors, including disease names/wound types, name of lumen site as a diseased part, degree, stage, etc., of a lesion or wound |
| Operation-Associated Information | Recommended actions, inserting instructions, warnings or alarms, ... caution points for operations |

Such types of operation support information are required to be conveyed to doctors as operators for easy comprehension in order that they can respond more quickly. Accordingly, not only the contents of the information, but also the communication methods are important, and it is necessary to process/convert the information into a plain form or consider a better outputting manner.

Note that the operation support information for recommended actions is intended for all the operations relating to the insertion unit, such as water supply, air supply, air intake, etc., in addition to the insertion and extraction operations and bending operations of the insertion unit as shown in FIG. 3. Any type of information may be adopted as this support information as long as the insertability, operability, etc., including the certainty and safety are improved.

Next, the storage 6 will be described.

With the storage 6, various types of information which have been acquired, extracted, or generated, are stored and read. Further, information required for each processing is stored in advance and read as appropriate and as needed.

In the present embodiment, the storage subjects include the following information.
1) Information obtained from each information acquirer
2) Information extracted or generated at the support information generator, and intermediate information thereof
3) Programs for generating support information, settings to prescribe what support information can be generated, etc., criteria for determining a particular state, etc.

Next, the display 16 will be described.

The display of the insertion unit support system serves also as the display 16 of the endoscope system (tubular insertion system) 100, and outputs the operation support information by displaying it in a superimposed manner on the endoscope image or alone on part of the display space. The output form may be a mere screen display by the display 16, but a sound, vibration, etc., may also be used for notification so that operators such as doctors can become promptly and easily informed. Moreover, the screen display may be combined with a sound, vibration, etc. The display 16 may also utilize not only a fixed monitor, but also wearable, portable display devices such as a head-mounted display. In this case, a sound may be given through earphones.

Relating to a mode for display, the operation support information is categorized into information to be constantly displayed and information to be displayed only when a particular condition is met. Depending on the output setting for the support information, an operator may decide the manner to output desired information or the insertion unit support system 1 may set it according to the skill, etc., of an operator. It is also possible to set a display manner so that no operation support information will be output until a particular condition is met, in accordance with the operator's desire (setting).

With the insertion unit support system 1 according to the present embodiment described above, the following effects can be provided.

By using the subject information based on the pre-acquired imaging device information and the sensor's insertion unit information for generating the operation support information, information about both the subject 5 and the insertion unit is made available at the time of insertion and operation of the insertion unit. Thus, as compared to the cases of only the subject information or the insertion unit information, the operability of the insertion unit is improved. Consequently, the insertability and the workability in observation/diagnosis, etc., are improved.

Further, by referring to the three-dimensional images by a CT apparatus, an MRI apparatus, etc., that give a perspective view of the inside of the subject 5 from a specific external direction or viewpoint, it is possible to accurately comprehend the shape and location of the lumen, conditions of a lesion, etc., inside the subject. Thus, the subject information can be obtained with a high accuracy and in a large amount.

Also, if an attempt to realize the same function were made at the time of insertion of the insertion unit 20 from the combination of the imaging device 11, i.e., a large device such as the CT apparatus 51 and the MRI apparatus 61, and the sensors, the insertion operations for the insertion unit 20 would involve extensive works and lead to constraints regarding available facilities. Depending on the imaging device 11, a subject patient could also be exposed to X-rays, an intensive electromagnetic field, etc. for a long time, or the subject information may not be obtained in real time. Therefore, with the configuration of acquiring the imaging device information in advance of endoscope observation, the imaging device information can be acquired in an optimum environment, and the necessary processing can be completed beforehand as much as possible. Accordingly, the accuracy of the imaging device information can be enhanced, and the information processing load at the time of inserting the insertion unit can be reduced, thereby enabling fast processing and introduction of inexpensive processing systems.

Moreover, as discussed above, when an observation target is a lumen of the subject 5 that can vary in shape, such as a large bowel, it is not easy to insert the insertion unit up to the targeted site or operate it based on the assumption of the shape, since the inside of the subject 5 is not visually observable from the outside in a direct manner. To address this, by using the subject information and the sensor information, it is possible to grasp characteristics including, for example, the shape and location of the lumen, the shape and arrangement of the insertion unit 20, and the position, distribution, type, etc., of a specific site. Thus, during the insertion and operation of the insertion unit 20, to-be-checked status information about the lumen and the insertion unit 20 can be reduced, or the certainty of the status information can be improved, thereby improving the insertability and operability of the insertion unit 20. Similar to the cases of lumens that can vary in shape, the same effect can be attained for the lumens that largely differ according to individual differences or personal differences.

The above described subject information and operation support information include the lumen shape and location information or secondary lumen shape and location information related to the shape or location of the lumen of the subject 5. As this lumen shape and location information, externally-acquired lumen shape and location information and its secondary information, as well as lumen shape and location information constructed based on the sensor information are available. Furthermore, information obtained through correction or addition to the externally-acquired lumen shape and location information or its secondary information, using the lumen shape and location information constructed based on the sensor information, is also available. By adopting such lumen shape and location information obtained from the imaging device 11 as the operation support information, it is possible to learn how to insert the insertion unit or how deep the insertion unit has been inserted more accurately than in the absence of the information. Also, by using the secondary information or the information corrected or added based on the sensor information, it is possible to obtain the lumen information of a subject that better fits the system or that is more accurate. As a result, the insertion unit's insertability and operability are improved.

The information associated with the generation of the operation support information for the insertion unit 20 includes preliminary imaging device information, pickup image information, sensor information, external information, subject or operator information, insertion unit or system configuration information, system setting, operator's instruction and assessment, etc. Based on these types of information, the insertion unit information, subject information, and further the operation support information are extracted or generated.

Additionally, by having the storage 6 in the architecture, it is possible to store desired information or all of these types of information as appropriate, and to read out necessary information at the necessary timing. By using the storage 6 to constitute a database of the information, it is possible to present the past support information at any timing. This allows for the advance confirmation of approximately what conditions the subject or the lumen is in, how it has been changed, what state it would become in the next insertion, how the insertion and the operation should be performed, and so on, before the insertion of the insertion unit. Storing the information as big data can open the door to extraction of beneficial information at a later stage, too.

The workability accompanying the insertability will be significantly improved by the support information including the work-associated information for works based on the secondary lumen shape and location information, secondary specific-site information, and the insertion unit's shape and arrangement. For example, the work-associated information includes work instructions, instructions for insertion and extraction operations in conjunction with the lumen information, cautions about passing through a narrowed portion or deviating from a route, and warning/avoidance instructions about a danger.

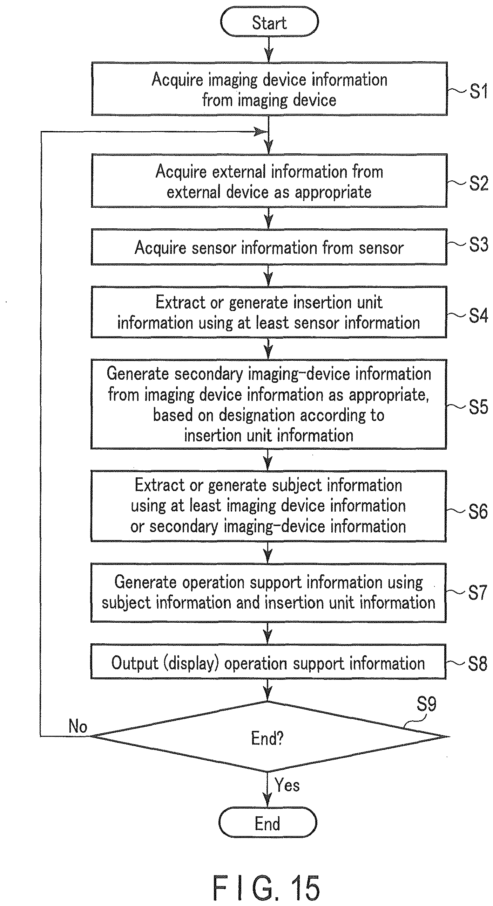

Next, referring to the flowchart shown in FIG. 15, the generation and output of the operation support information in the insertion unit support system 1 according to the embodiment will be described.

Initially, as a preliminary step, the endoscope 13 and the insertion unit support system 1 are connected with each other, and whether or not operation support is applicable is determined based on the ID tag 37. If it is determined that support can be provided, setting and initialization for operation support are performed. Then, the preliminary imaging device information including any of the CT image information, MRI image information, or ultrasonic image information is imported from the imaging device 11 into the subject information extractor 2 via the imaging-device information acquirer 10 (step S1).

Subsequently, and as needed, the external information is imported from external devices, etc. (not shown) into the insertion-unit information extractor 3 via the external information acquirer 9 (step S2). According to the setting, the external information is acquired for only the amount that is necessary. In particular, if no external information is required, this step S2 is skipped. Further, the above described sensor information detected by the shape sensor 15, the insertion unit sensor 86, and the operational amount sensor, provided for the endoscope 13, are imported into the insertion-unit information extractor 3 via the sensor information acquirer 8 (step S3).

Next, the insertion-unit information extractor 3 extracts or generates the insertion unit information using at least the sensor information among the imported information. The insertion unit information is sent to the subject information extractor 2 and, if necessary, to the support information generator 4 (step S4). The subject information extractor 2 generates the secondary imaging-device information from the imaging device information based on the input designation according to the insertion unit information (step S5), and further extracts or generates the subject information using at least one of the imaging device information and the secondary imaging-device information and sends it to the support information generator 4 (step S6).

The support information generator 4 generates the operation support information using the subject information from the subject information extractor 2 and the insertion unit information from the insertion-unit information extractor 3 (step S7). The generated operation support information is output to operators via the display 16, etc. using a screen display, sound, vibration, etc. (step S8). Thereafter, whether or not the set operation support has been finished is determined (step S9). If the support has not been finished (NO), the processing flow returns to step S2 and information acquisition from the external devices will be performed again. If the support has been finished (YES), the insertion support routine ends.

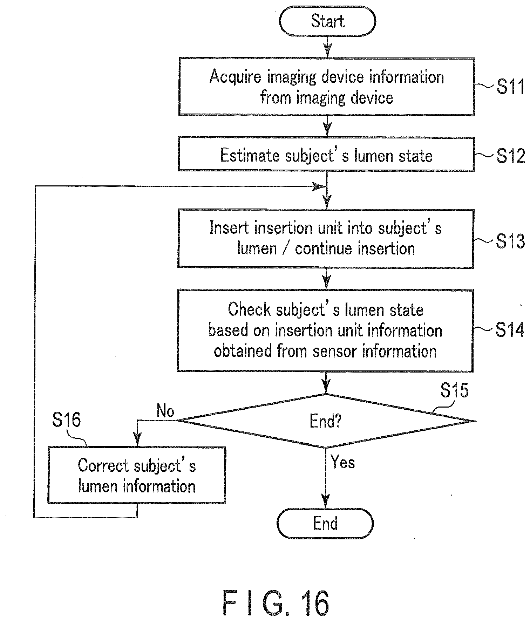

Next, referring to the flowchart shown in FIG. 16, the processing to correct or update the information about the subject's lumen state will be described.

First, the imaging device information including a three-dimensional image or a three-dimensional tomogram is imported into the subject information extractor 2 via the imaging-device information acquirer 10 (step S11). Using the routine in FIG. 15 described above, the lumen state (shape, location, etc.) of the subject 5 is estimated (step S12). An operator grips the endoscope 13 and inserts the insertion unit 20 into the lumen of the subject 5 (step S13). At this time, the sensor information is acquired from the sensor portion 28 mounted at the endoscope 13 to generate information about the actual lumen state of the subject 5, and whether or not a correction is required is checked by comparing the estimated lumen state with the actual lumen state of the subject 5 (step S14). Next, whether or not the estimated lumen state is proper so that no correction is required and the processing may end is determined (step S15). If no correction is required for the estimated lumen state (YES), the estimated lumen state is output to the support information generator 4 and the processing ends. On the other hand, if there is a difference between the estimated lumen state and the actual lumen state of the subject 5 and the estimated lumen state requires a correction (NO), the estimated lumen information is corrected based on the sensor information (step S16), and the processing flow returns to step S13 to continue with the insertion of the insertion unit into the lumen of the subject 5 again.

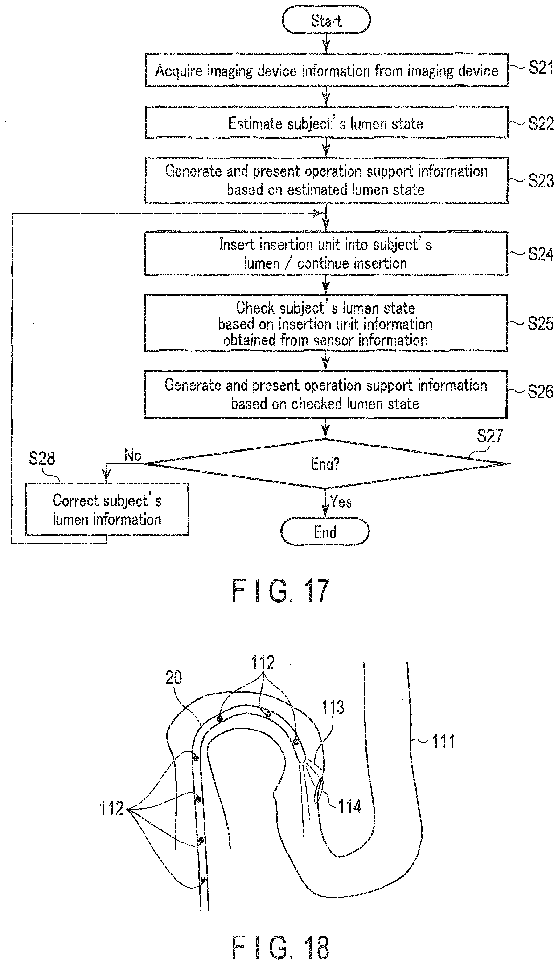

Next, referring to the flowchart shown in FIG. 17, the support information processing for presenting the subject's estimated lumen state and the subject's measured lumen state will be described.

First, the imaging device information including a three-dimensional image or a three-dimensional tomogram is imported into the subject information extractor 2 via the imaging-device information acquirer 10 (step S21). Using the routine in FIG. 15 described above, the lumen state (shape, location, etc.) of the subject 5 is estimated (step S22), and the operation support information based on the estimated lumen state is generated and displayed on the display 16 in the form of an image (step S23).

Next, an operator grips the endoscope 13 and inserts the insertion unit 20 into the lumen of the subject 5 (step S24). At this time, the updated sensor information from the sensor portion 28 mounted at the endoscope 13 is acquired, and the actual lumen state of the subject 5 is checked based on the sensor information (step S25). After this check, the support information generator 4 generates the operation support information based on the actual lumen state of the subject 5, and the display 16 is caused to display it in the form of an image (step S26). Then, whether or not a correction is required and the processing may end is determined by comparing the operation support information based on the estimated lumen state with the operation support information based on the actual lumen state of the subject 5 (step S27). If no correction is required, the processing ends. On the other hand, if there is a difference between the estimated lumen state and the actual lumen state of the subject 5 and the estimated lumen state requires a correction (NO), the estimated lumen information is corrected based on the sensor information (step S28), and the processing flow returns to step S24 to continue with the insertion of the insertion unit into the lumen of the subject 5 again.

FIG. 18 shows a certain example of the operation support information displayed on the display 16.

This operation support information is indicative of a state where the endoscope 13 is inserted into the large bowel of the subject 5. Normally, the insertion state within a subject cannot be directly comprehended except when pickup images taken by the endoscope 13 are given. According to this embodiment, the following information serves as the operation support information at the time of inserting the insertion unit 20 of the endoscope 13.
1) Information about the location and the shape of the large bowel 111 in the subject 5 - generated using part of the imaging device information from the imaging device 11
2) Information about the bending form of the endoscope's insertion unit 20 - generated based on the sensor information acquired from multiple sensors 112
3) Information about the arrangement and the shape of the endoscope's insertion unit 20 in the large bowel 111 - generated from the combination of 1) and 2)
4) Information about the large bowel 111 and a lesion part 114 within the range (observation field or imaging field) illuminated by the endoscope's illumination light 113 - generated from the combination of 1) and 2)

Next, examples of the insertion-state detection according to the operation support information displayed on the screen of the display 16 will be described.

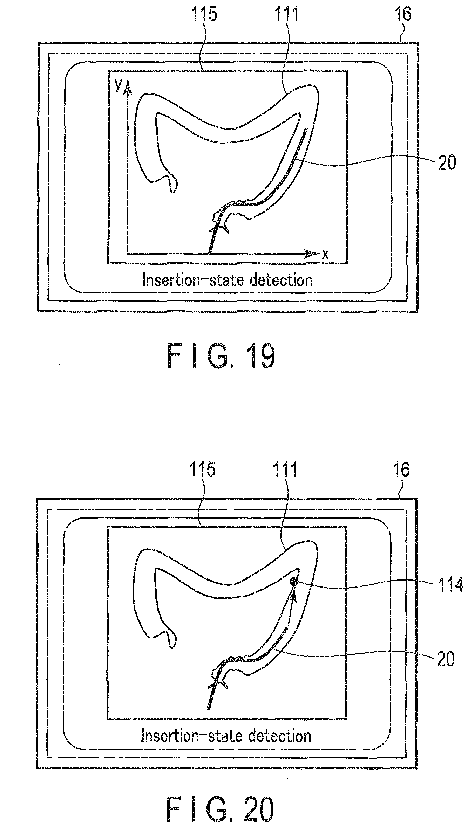

FIG. 19 is the screen that displays, as a first display example, the insertion-state detection indicative of the oriented distal end of the insertion unit 20 of the endoscope 13 that will form an arrangement relationship with a lesion part as a specific site.

As the operation support information for this display example, 1) the information about the location and the shape of the large bowel 111, 2) the information about the arrangement and the shape of the insertion unit 20, and 3) the information about the position of the distal end of the insertion unit 20 and its imaging direction are displayed.

FIG. 20 is the screen that displays, as a second display example, the insertion-state detection indicative of the arrangement relationship between the oriented distal end of the insertion unit 20 of the endoscope 13 and the lesion part 114 as a specific site.

As the operation support information for this display example, the forward direction of the distal end of the insertion unit 20 toward the lesion part, the distance from the distal end to the lesion part, and instructions about the endoscope's insertion operation for the distal end to reach the lesion part are displayed in addition to the position relationship between the large bowel 111 and the insertion unit 20, and they are updated moment by moment. Thereby, reaching the lesion part becomes very easy.

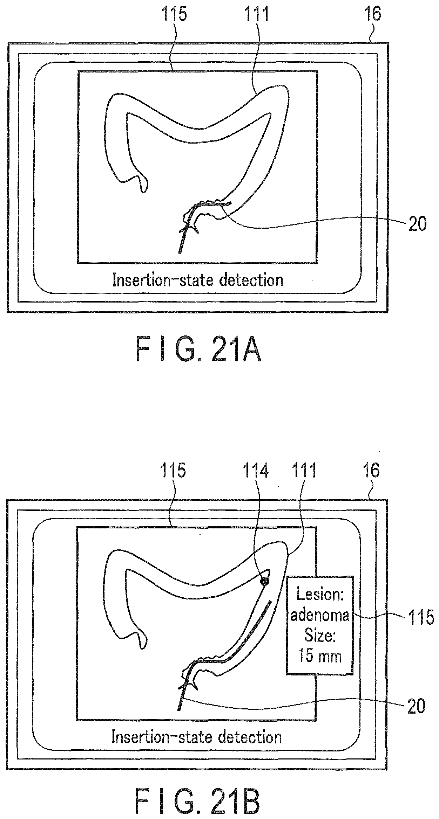

FIGS. 21A and 21B show, as a third display example, the example in which the display and non-display of the operation support information are switched based on the proximity relationship between the distal end of the insertion unit 20 and a specific site.

In FIG. 21A, the distal end of the insertion unit 20 is distant from the specific site, and accordingly, only the position of the distal end within the large bowel 111 is displayed and information about the specific site is not displayed. When the insertion unit 20 is inserted further in the large bowel 111, and the distal end of the insertion unit 20 has reached a position at which the distance shown in FIG. 21B becomes a certain distance or smaller, the type of the lesion part (lesion: adenoma) and its size (size: 15 mm) are displayed as lesion information 115 from then on. Also, if there are a number of specific sites in the large bowel 111 and each of them is required to be checked, the positions of the respective lesion parts are all displayed, and only the lesion information for the lesion part that has come to a position at a certain or smaller distance from the distal end or the lesion part that is closest to the distal end is displayed. Thereby, the operational efficiency of the operator is improved.

Moreover, the output state/output contents of the operation support information, such as the orientation of the distal end of the insertion unit 20 and the distance from the distal end, are changed based on the relationship between the distal end of the insertion unit 20 and the specific site. For example, the display may be started upon approximation to a position at a certain distance, and terminated upon separation with a certain or larger distance. In this example, the display is given only when the orientation of the distal end of the insertion unit 20 substantially conforms to the direction toward the specific site. By switching the display contents according to the particulars of the specific site, the state of operation support can be suited to the approximated specific site, and providing unnecessary information can be avoided.

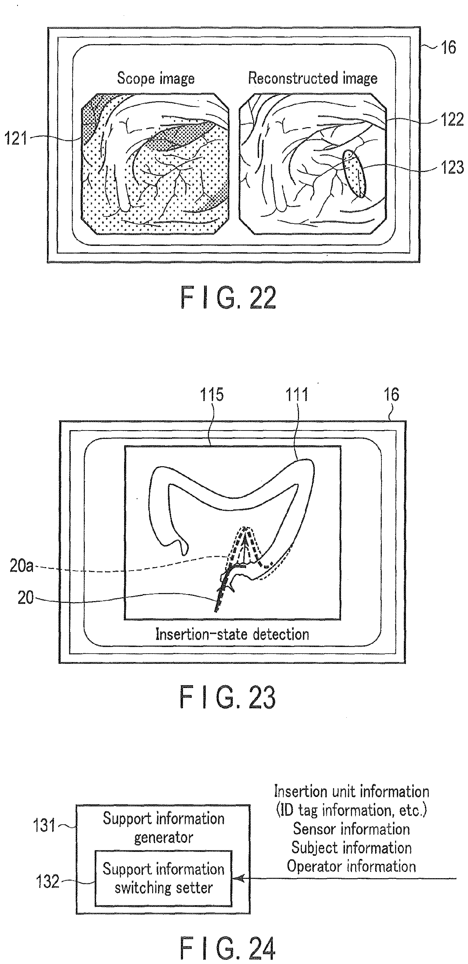

Next, FIG. 22 shows an example where an endoscope's pickup image (scope image 121) and a reconstructed image formed of the secondary imaging-device information generated from the imaging device information are displayed side by side on the screen of the display 16. For this display, the reconstructed image 122 as the secondary device information has been produced from the imaging device information, following the designation according to the insertion unit information, so that the reconstructed image 122 substantially conforms to the scope image 121 taken by the endoscope 13 in viewpoint and screen size. The reconstructed image 122 shows a site 123 included in the imaging device information (or external information) and suspected of a lesion in a superimposed manner on the region targeted for observation or treatment. In the reconstructed image 122, the lesion suspected site 123 is marked. With such image processing, e.g., using a brighter color than the surrounding area, for displaying the suspected site to stand out as an observation or treatment target region or as an area including the target site, operators of the insertion unit 20 can instantly pay attention to the support information displayed in a distinctive manner.

The insertion unit information, including the position of the distal end of the insertion unit 20 with respect to the large bowel, the orientation of its imager's viewpoint, etc., can be obtained based of the estimation from the scope image 121 or the sensor information. By clearly indicating the observation/treatment target region on the screen together with the endoscope's pickup image in this manner, it is possible to guide the distal end of the insertion unit 20 to the observation target site without an oversight.

In addition, even after the observation/treatment target region has been actually confirmed by an endoscope, the current location of the target region could change or the target region could be blocked from discovery, the presence of another untouched site could be confirmed, or the lesion condition may differ from that assumed. In that case, correcting/updating the subject information can contribute to the observation and treatment at the next endoscope insertion. The causes shown in Table 5 are conceivable as the reasons for performing such correction or update.

**[Table 5]**

| | |
|---|---|
| Reasons for Update | Aging variation, implementation of detection using better sensitivity/accuracy, changes due to insertion of the insertion unit |
| Contents of Update | Updating the location and shape of the lumen |
| | Updating the location, shape, position, and range of the specific site |
| | Updating/correcting the examination or diagnosis result |

FIG. 23 shows an example of a case where the large bowel 111 is deformed by the inserted insertion unit 20. When the insertion unit 20 of the endoscope 13 is inserted, the shape of the movable large bowel 111 could be changed, or the shape of the large bowel 111 could differ from the shape that has been generated based on the pre-acquired imaging device information.

Accordingly, when information is acquired that is indicative of a large deformation of the large bowel 111 due to the insertion of the insertion unit 20, a warning is issued to indicate that the insertion unit 20 has deviated from the intended insertion route. Further, when the large bowel 111 is under pressure, a warning is issued to appropriately prompt an instruction to immediately stop the insertion operation of the insertion unit 20 or to retract the insertion unit 20. By issuing such warning guidance, it is possible to prevent the insertion in wrong directions so that the insertion up to the observation target site is facilitated, the insertion time is reduced, and the load on patients as well as the large bowel and other organs as lumens can be reduced.

When the shape of the large bowel 111, acquired as the sensor information, is different from the shape obtained based on the pre-acquired imaging device information, the subject information is corrected so that it conforms to the current shape and location of the large bowel. Alternatively, the insertion route is corrected by setting a new insertion route for the insertion unit 20 so that it accords with the current shape and location of the large bowel. In this manner, correcting/updating the shape and location information of the large bowel 111 and the insertion route can facilitate the next insertion operation of the insertion unit 20.

Next, referring to FIG. 24, an example of the support information and how to generate the support information will be described.

As shown in FIG. 2 described earlier, the ID tag 37 is attached above the operation portion 30 for the insertion unit 20 of the endoscope 13. The ID tag information of this ID tag 37 includes a model type of the endoscope, equipping options, etc. The equipping options include, for example, sensor types and configurations if sensors are incorporated. Furthermore, the information may together include functions available for the endoscope 13, product states such as a normal/abnormal state, and maintenance history. Also, in addition to such information, the ID tag 37 may describe information about endoscope specifications and equipped optional devices and sensors, as well as the identification information such as a model number.

The ID tag information as such additionally includes information as to whether or not the insertion unit support system 1 is applicable, and function information available for support. When the endoscope 13 is connected to the insertion unit support system 1 electrically, etc., the ID tag 37 is read so that the support contents suitable for the system can be set based on the ID tag information. For example, the endoscope 13 may be of various types, such as an old or a new model, one including a slender or a large insertion unit, one including an easily-bendable distal end of the insertion unit, or one capable of adjusting the rigidity of the insertion unit. The operation support suitable to each of these types can be set.

Such switching of the support information contents and providing methods is made possible by furnishing the support information generator 131 of the insertion unit support system 1 with a support information switching setter 132 as shown in FIG. 24. In this example, the support information switching setter 132 is provided within the support information generator 131, but it may be provided at any position within the insertion unit support system 1 without limitation. In this manner, it is possible to present the points of insertion operations, how to address the difficulties at the time of insertion, and so on, as the optimal support information according to the characteristics and specifications of each type of the endoscope 13. Thereby, the workload and anxiety of an operator can be largely removed.

### REFERENCE SIGNS LIST

1 ... insertion unit support system, 2 ... subject information extractor, 3 ... insertion-unit information extractor, 4 ... support information generator, 5 ... subject, 6 ... storage, 7 ... pickup information acquirer, 8 ... sensor information acquirer, 9 ... external information acquirer, 10 ... imaging-device information acquirer, 11 ... imaging device, 12 ... secondary imaging-device information generator, 13 ... endoscope, 14 ... image processor, 15 ... shape sensor, 16 ... display (monitor), 18 ... light source, 19 ... Controller, 20 ... insertion unit, 21 ... distal rigid portion, 23 ... bending portion, 25 ... flexible tube portion, 27 ... universal cable, 28 ... sensor portion, 30 ... operation portion, 31 ... grip portion, 34 ... operation buttons, 35 ... insertion port, 36 ... bending operation portion, 37 ... ID tag, 38 ... detection subject portion, 39 ... imager, 51 ... CT apparatus, 55 ... imager, 56 ... collimater, 57 ... image information, 58 ... helical scanning, 61 ... MRI apparatus, 65 ... ultrasonic diagnosis apparatus, 66 ... apparatus body, 67 ... probe, 67a ... convex-type probe, 67b ... linear-type probe, 68 ... monitor, 69 ... caster rack, 70 ... doctor, 71 ... sensor main body, 72 ... optical fiber, 73 ... reflector, 74 ... shape calculator, 75 ... controller, 76 ... light source, 77 ... projector lens, 78 ... isolator, 79 ... reflective mirror, 80 ... condenser lens, 81 ... second condenser lens, 82 ... optical detector, 85 ... oral cavity, 86 ... insertion unit sensor, 91 ... lumen information integrating processor, 92 ... position information integrating processor, 93 ... lumen-associated information estimate generator, 94 ... lumen location-associated information estimate generator, 100 ... endoscope system, 111 ... large bowel, 112 ... sensor, 113 ... illumination light, 114 ... lesion part, 115 ... lesion information, 121 ... scope image, 122 ... reconstructed image, 123 ... site, 131 ... support information generator, 132 ... support information switching setter, A1 ... forward/backward movement amount, A2 ... twist amount, A3 ... up/down direction, A4 ... left/right direction

## Claims

1. An insertion unit support system configured to provide support information for operations with an insertion unit adapted to be inserted into a given lumen of a subject, wherein the operations comprise insertion and extraction of the insertion unit, observation, and treatment, and wherein the support information is based on pre-acquired lumen information comprising two- or higher dimensional shape information for the lumen.

2. The insertion unit support system according to claim 1, comprising:
an imaging-device information acquirer configured to acquire imaging device information comprising the lumen information for the given lumen of the subject, wherein the lumen information is pre-acquired by an imaging device configured to acquire two- or higher dimensional image information;
a sensor information acquirer configured to acquire sensor information obtained by a sensor configured to detect a state of the insertion unit inserted into the lumen;
a subject information extractor configured to extract or generate subject information from the imaging device information;
an insertion-unit information extractor configured to extract or generate insertion unit information from the sensor information;
a support information generator configured to generate operation support information from the subject information and the insertion unit information for supporting an operator in endoscope insertion and endoscope operations; and
an outputter configured to output the operation support information.

3. The insertion unit support system according to claim 1, wherein the given lumen of the subject changes a shape according to insertion of the insertion unit.

4. The insertion unit support system according to claim 2, wherein the subject information comprises part of the imaging device information acquired from an imaging device configured to take a three-dimensional image that gives a perspective view of an inside of the subject from a specific external direction or viewpoint, or secondary imaging-device information obtained based on the image information.

5. The insertion unit support system according to claim 4, wherein the secondary imaging-device information comprises secondary image information as reconstructed image information based on a specific viewpoint/cross-section.

6. The insertion unit support system according to claim 4, wherein the secondary imaging-device information comprises secondary lumen shape and location information indicative of a shape or a location of the lumen based on a specific viewpoint/cross-section.

7. The insertion unit support system according to any one of claims 4 to 6, wherein the imaging device information or the secondary imaging-device information further comprises specific-site information for a specific site which is a site requiring attention in the insertion and extraction of the insertion unit or a site targeted for observation/diagnosis or treatment operations.

8. The insertion unit support system according to any one of claims 4 to 7, wherein the subject information extractor comprises a secondary imaging-device information generator configured to generate the secondary imaging-device information.

9. The insertion unit support system according to claim 4, wherein the insertion unit comprises an imager configured to take an image inside the lumen, and the subject information and the operation support information comprise the imaging device information or the secondary imaging-device information that corresponds to the image of the lumen obtained from the imager.

10. The insertion unit support system according to claim 9, wherein the subject information and the operation support information further comprise secondary specific-site information that corresponds to a specific site which is a site requiring attention in the insertion and extraction of the insertion unit or a site targeted for observation/diagnosis or treatment operations.

11. The insertion unit support system according to claim 4, wherein the subject information and the operation support information comprise lumen shape and location information or secondary lumen shape and location information for a shape and location of the lumen.

12. The insertion unit support system according to claim 11, wherein the subject information and the operation support information further comprise specific-site information that corresponds to a specific site which is a site requiring attention in the insertion and extraction of the insertion unit or a site targeted for observation/diagnosis or treatment operations.

13. The insertion unit support system according to claim 2, wherein the subject information extractor comprises a lumen information integrating processor configured to integrate the imaging device information and information that is related to the lumen and included in the sensor information.

14. The insertion unit support system according to claim 13, wherein the lumen information integrating processor comprises a position information integrating processor configured to integrate the imaging device information and position information that is included in the sensor information.

15. The insertion unit support system according to claim 14, wherein the position information integrating processor generates a position coordinate for the lumen in order to generate or present the support information, through interpolation or estimation.

16. The insertion unit support system according to claim 14, wherein the position information integrating processor comprises a lumen location-associated information estimate generator configured to generate current lumen location-associated information through estimation, in order to generate or present the support information when there is a difference or a missing portion between the lumen information based on the imaging device information and lumen location-associated information based on the sensor information in portions sharing any of a shape, size, position, and orientation.

17. The insertion unit support system according to claim 16, wherein the lumen location-associated information estimate generator generates the current lumen location-associated information based on an assumption that the position information included in the sensor information is correct.

18. The insertion unit support system according to claim 4, further comprising an imager configured to image the lumen of the subject to obtain a pickup image information, near a distal end of the insertion unit, wherein the insertion-unit information extractor corrects or updates the subject information based on at least one of the pickup image information and the sensor information.

19. The insertion unit support system according to claim 18, wherein
the sensor is configured to detect/estimate at least one of a shape, arrangement, and orientation of the insertion unit, or distal end information comprising a position, direction, and orientation of the distal end,
the subject information comprises lumen shape and location information for a shape and location of the lumen,
the insertion-unit information extractor generates, as at least part of the insertion unit information, insertion-unit shape and arrangement information for a shape and arrangement of the insertion unit based on the sensor information obtained from the sensor, and
the subject information is corrected or updated based on the lumen shape and location information and the insertion-unit shape and arrangement information.

20. The insertion unit support system according to claim 18, wherein the subject information is corrected or updated on a real-time basis.

21. The insertion unit support system according to claim 4, wherein
the sensor is configured to detect/estimate at least one of arrangement information comprising a shape and orientation of the insertion unit, or distal end information comprising a position, direction, and orientation of a distal end of the insertion unit, and
the operation support information is generated from the insertion unit information based on the sensor information obtained from the sensor and the subject information based on the imaging device information.

22. The insertion unit support system according to claim 21, further comprising an imager configured to image the lumen of the subject to obtain a pickup image information, near a distal end of the insertion unit,
wherein the secondary imaging-device information corresponding to the imaging device information is generated based on shape and arrangement information of the insertion unit, or the distal end information and the imaging device information.

23. The insertion unit support system according to claim 21, wherein
the secondary imaging-device information comprises secondary lumen shape and location information indicative of a shape and location of the lumen based on a specific viewpoint/cross-section,
the insertion unit information comprises shape and arrangement information of the insertion unit based on the specific viewpoint/cross-section, and
the operation support information comprises image information based on a combination of the secondary lumen shape and location information and the shape and arrangement information of the insertion unit.

24. The insertion unit support system according to claim 21, wherein the operation support information comprises support information comprising a direction and distance from the distal end of the insertion unit, which are based on an arrangement relationship between a specific-site and the insertion unit, the specific site being a site requiring attention in the insertion and extraction of the insertion unit or a site targeted for observation/diagnosis or treatment operations.

25. The insertion unit support system according to claim 21, wherein output state/output contents of the operation support information comprising a direction and distance from the distal end of the insertion unit are changed based on a distance or an arrangement relationship between a specific-site and the insertion unit with respect to an observation/operation-allowable range, the specific site being a site requiring attention in the insertion and extraction of the insertion unit or a site targeted for observation/diagnosis or treatment operations.

26. The insertion unit support system according to claim 21, wherein
the secondary imaging-device information comprises secondary lumen shape and location information indicative of a shape/location of the lumen based on a specific viewpoint/cross-section, and
the operation support information comprises insertion and extraction operation-associated information for an operation of the insertion and extraction of the insertion unit based on secondary lumen shape and location information and a shape and arrangement of the insertion unit.

27. The insertion unit support system according to claim 21, wherein the secondary imaging-device information comprises
secondary lumen shape and location information indicative of a shape/location of the lumen based on a specific viewpoint/cross-section, and
secondary specific-site information for a specific site as a site requiring attention in the insertion and extraction of the insertion unit or a site targeted for observation/diagnosis or treatment operations, and
the operation support information comprises work-associated information for works based on the secondary lumen shape and location information, the secondary specific-site information, and a shape and arrangement of the insertion unit.

28. The insertion unit support system according to claim 2, comprising a storage configured to enable storage or readout of at least part of acquired, extracted, or generated information, and/or to store information for generating the operation support information beforehand and enable readout as needed.

29. The insertion unit support system according to claim 1, wherein the insertion unit comprises an ID tag comprising at least one of identification number, specification information, and status information of the insertion unit, and
the insertion unit support system identifies the support information to provide based on the at least one information.

30. The insertion unit support system according to claim 29, wherein setting for generating the support information to provide is performed based on the at least one information from the ID tag.

31. The insertion unit support system according to claim 29, which identifies the support information to provide upon activation or upon connection or removal of a system component.
